# EUROPEAN PATENT APPLICATION

(11) **EP 4 772 173 A1**
(43) Date of publication of application: **08.07.2026**
(21) Application number: 23950166.1
(22) Date of filing: 31.08.2023
(51) Int. Cl.: A61K 9/127, A61K 31/4745, A61K 47/34, A61P 35/00

(54) **IRINOTECAN HYDROCHLORIDE LIPOSOME AND PREPARATION METHOD THEREFOR**

(71) Applicant: Sichuan Kelun Pharmaceutical Research Institute Co., Ltd., Chengdu, Sichuan 611138 (CN)
(72) Inventor: HAO, Fei, Chengdu, Sichuan 611138 (CN); SU, Zhengxing, Chengdu, Sichuan 611138 (CN); ZHAO, Jinlong, Chengdu, Sichuan 611138 (CN); LI, Ming, Chengdu, Sichuan 611138 (CN); BAO, Fei, Chengdu, Sichuan 611138 (CN); ZHAO, Dong, Chengdu, Sichuan 611138 (CN)
(74) Representative: Hoffmann Eitle
(86) International application number: PCT/CN2023/116026
(87) International publication number: WO 2025/043579

(57) **Abstract**

Provided in the present invention is an irinotecan hydrochloride liposome composition, comprising the following components in a specific weight ratio: irinotecan hydrochloride, hydrogenated soybean phospholipid, cholesterol and mPEG2000-DSPE. The present invention also provides a preparation method for said liposome composition. The liposome of the present invention can significantly reduce the severe blood toxicity of irinotecan hydrochloride, and especially significantly reduce the incidence of neutropenia.

## Description

### TECHNICAL FIELD

The present invention relates to the field of pharmaceutical formulations. More specifically, the present invention relates to a liposomal composition of irinotecan hydrochloride and a preparation method therefor.

### BACKGROUND ART

Irinotecan hydrochloride is a camptothecin derivative with proven efficacy and broad antitumor activity. Currently, commercially available formulations of irinotecan hydrochloride include conventional injections and liposomal formulations. The conventional injection is composed of lactic acid, sorbitol, and water for injection. As the drug is photosensitive, it is packaged in brown glass vials and is primarily used as monotherapy or in combination therapy for the treatment of advanced colorectal cancer. The liposomal formulation utilizes polyanionic sucrose octasulphate to encapsulate irinotecan and is applied in combination with 5-fluorouracil and leucovorin for second-line treatment of metastatic pancreatic cancer with disease progression.

Administration of the conventional irinotecan hydrochloride injection causes severe, life-threatening toxic side effects such as myelosuppression and diarrhea, *etc*. These drug toxicities limit its clinical therapeutic application. Liposomes encapsulate irinotecan within a sealed vesicle cavity, protecting it from hydrolytic ring-opening. They also confer long-circulating properties, prolonging the half-life of irinotecan, improving its tissue distribution, increasing accumulation at tumor sites, and enhancing antitumor efficacy. However, irinotecan liposomes prepared according to prior art still face the challenge of severe, fatal, and life-threatening neutropenic hematological toxicity. For example, ONIVYDE^{®}, approved in 2015, still lists severe, fatal neutropenia as a key warning in the FDA's safety black box. New liposomal formulations of irinotecan with reduced toxicity remain an urgent clinical need.

The hematological toxicity of irinotecan primarily stems from the distribution and accumulation of its active metabolite, SN-38, in the bone marrow. SN-38 in the bone marrow mainly originates from plasma SN-38 and from the metabolism of irinotecan distributed therein. Minimizing the distribution and accumulation of SN-38 in bone marrow can effectively reduce drug-induced hematological toxicity.

### CONTENTS OF THE INVENTION

The present invention aims to provide a liposome composition and formulation of irinotecan hydrochloride with lower toxicity, as well as the method for their preparation. It has been unexpectedly found that by strictly controlling the ratio of the drug irinotecan hydrochloride to phospholipid, the formulation composition ratios of the liposomes, the composition and concentration of the internal aqueous phase, and the preparation methods (particularly the mass ratio of phospholipid to ethanol, the volume ratio of the aqueous phase to the ethanol phase, and the method of incorporating the long-circulating material), the bone marrow distribution and release characteristics of irinotecan liposomes in vivo can be significantly optimized, thereby achieving the objective of markedly reducing hematological toxicity.

The liposomal composition of irinotecan hydrochloride provided by the present invention, when administered to patients with advanced or metastatic solid tumors, can significantly reduce the severe hematological toxicity of irinotecan hydrochloride, particularly the incidence of neutropenia, and markedly improve patient survival status.

To achieve the above objectives, the present invention provides a liposomal composition of irinotecan hydrochloride, characterized in that the liposomal composition comprises components in the following mass ratios: 1 part of irinotecan hydrochloride, 3.67-5.62 parts of hydrogenated soybean phospholipids, 1.23-2.25 parts of cholesterol, and 0.055-0.200 part (preferably 0.060-0.200 part, preferably 0.070-0.200 part, even more preferably 0.0801-0.200 part) of methoxy-terminated poly(ethylene glycol) 2000 distearoylphosphatidylethanolamine (mPEG2000-DSPE); the weight ratio of the hydrogenated soybean phospholipids to cholesterol is 2-4.5:1.

In some embodiments of the present invention, the liposomal composition comprises components in the following mass ratios: 1 part of irinotecan hydrochloride, 4.13-5.05 parts of hydrogenated soybean phospholipids, 1.23-1.86 parts of cholesterol, and 0.0801-0.150 part of methoxy-terminated poly(ethylene glycol) 2000 distearoylphosphatidylethanolamine (mPEG2000-DSPE); the weight ratio of the hydrogenated soybean phospholipids to cholesterol is 2-3.5:1.

In some embodiments of the present invention, the liposomal composition comprises components in the following mass ratios: 1 part of irinotecan hydrochloride, 4.13-5.05 parts of hydrogenated soybean phospholipids, 1.39-1.71 parts of cholesterol, and 0.0810-0.110 part of methoxy-terminated poly(ethylene glycol) 2000 distearoylphosphatidylethanolamine (mPEG2000-DSPE); the weight ratio of the hydrogenated soybean phospholipids to cholesterol is 2-3.5:1.

In some embodiments of the present invention, the liposomal composition comprises components in the following weight ratios: 1 part of irinotecan hydrochloride, 4.59 parts of hydrogenated soybean phospholipids, 1.5468 parts of cholesterol, and 0.0840 part of methoxy-terminated poly(ethylene glycol) 2000 distearoylphosphatidylethanolamine (mPEG2000-DSPE).

In some embodiments of the present invention, the liposomal composition comprises ammonium sulfate in an internal aqueous phase, and the concentration of ammonium sulfate in the internal aqueous phase is 0.16-0.28 M, preferably 0.20-0.24 M, and more preferably 0.22 M.

In some embodiments of the present invention, the liposomal composition of irinotecan hydrochloride is prepared by an ethanol injection method and an ammonium sulfate gradient method, wherein the mass ratio of hydrogenated soybean phospholipids to ethanol in the ethanol phase is 0.1:1-0.5:1 (preferably 0.1:1-0.35:1, more preferably 0.1:1-0.2:1), the concentration of ammonium sulfate in the aqueous phase is 0.16-0.28 M (preferably 0.20-0.24 M, more preferably 0.22 M), and the volume ratio of the ethanol phase to the aqueous phase is 1:3-1:6.

In some embodiments of the present invention, the liposomal composition of irinotecan hydrochloride is prepared by an ethanol injection method and an ammonium sulfate gradient method, wherein the mass ratio of hydrogenated soybean phospholipids to ethanol in the ethanol phase is 0.1:1-0.35:1, the concentration of ammonium sulfate in the aqueous phase is 0.20-0.24 M, and the volume ratio of the ethanol phase to the aqueous phase is 1:3-1:6.

In some embodiments of the present invention, the liposomal composition of irinotecan hydrochloride is prepared by an ethanol injection method and an ammonium sulfate gradient method, wherein the mass ratio of hydrogenated soybean phospholipids to ethanol in the ethanol phase is 0.1:1-0.2:1, the concentration of ammonium sulfate in the aqueous phase is 0.22 M, and the volume ratio of the ethanol phase to the aqueous phase is 1:3-1:6.

In some embodiments of the present invention, the liposomal composition has a particle size of 90-110 nm, and the particle size D90 corresponding to the cumulative particle size distribution reaching 90% is <300 nm, such as D90 <200 nm or D90 <150 nm.

In some embodiments of the present invention, the concentration of irinotecan hydrochloride in the liposomal composition is 4.25-5.75 mg/ml.

In some embodiments of the present invention, the concentration of irinotecan hydrochloride in the liposomal composition is 4.5-5.5 mg/ml.

In some embodiments of the present invention, the concentration of irinotecan hydrochloride in the liposomal composition is 5 mg/ml.

The present invention further provides use of ammonium sulfate as the internal aqueous phase in the liposomal composition of irinotecan hydrochloride of the present invention, wherein the concentration of ammonium sulfate in the internal aqueous phase is controlled at 0.16-0.28 M.

The present invention provides a method for preparing a liposomal composition of irinotecan, comprising the following steps:
a. preparing an ethanol phase: dissolving hydrogenated soybean phospholipids and cholesterol in absolute ethanol at 50-70°C, and maintaining it at the temperature for later use;
b. preparing an aqueous phase: dissolving ammonium sulfate in purified water at 50-70°C to obtain an ammonium sulfate solution with an ammonium sulfate concentration of 0.16-0.28 M (preferably 0.20-0.24 M, more preferably 0.22 M), and maintaining it at the temperature for later use;
c. injecting the ethanol phase into the aqueous phase at a volume ratio of 1:3-1:6, and processing the liposomes to 85-150 nm using an extrusion device;
d. removing ammonium sulfate from the external aqueous phase: preparing a phosphate buffer solution at pH 5.0-7.0 as a displacement medium to displace the external aqueous phase of the liposomes using a tangential flow ultrafiltration device, thereby forming an ammonium ion gradient difference of greater than 10³-fold between the internal and external aqueous phases of the liposomes;
e. drug loading: adding a mixed solution of irinotecan hydrochloride and mPEG2000-DSPE to the blank liposomes having the ammonium ion gradient difference, followed by incubation at 55-60°C for 5-30 minutes to obtain the liposomal composition of irinotecan hydrochloride.

In some embodiments of the present invention, the method further comprises steps of incubation, quenching, and sterilizing the resulting liposomal solution of irinotecan hydrochloride by filtration through a 0.2 µm filter membrane.

In some embodiments of the present invention, in step e, after the incubation is completed, ice-bath quenching is performed, the resulting liposomal solution of irinotecan hydrochloride is sterilized by filtration through a 0.2 µm filter membrane, and then filled under a nitrogen atmosphere into vials, and sealed.

In some embodiments of the present invention, the dissolution temperature in steps a and b is 60°C; and/or the volume ratio of the ethanol phase to the aqueous phase in step c is 1:5; and/or the phosphate buffer solution in step d is a 9% sucrose (w/v)-0.1 mol/L phosphate buffer solution with pH 6.5; and/or the drug loading time in step e is 10 minutes.

The present invention simultaneously utilizes functional materials to modify the liposomes during drug loading, imparting long-circulating properties. This approach neither affects the liposomal encapsulation efficiency nor additionally increases the high-temperature processing time for mPEG2000-DSPE during liposome preparation, facilitating the production of high-quality products.

In some embodiments of the present invention, the liposomal composition of irinotecan hydrochloride prepared by the preparation method has a liposome particle size of 90-110 nm, and the particle size D₉₀ corresponding to the cumulative particle size distribution reaching 90% is < 300 nm, preferably D₉₀ <200 nm, more preferably D₉₀ <150 nm.

In some embodiments of the present invention, the liposomal composition of irinotecan hydrochloride prepared by the preparation method has an irinotecan hydrochloride concentration of 4.25-5.75 mg/ml, preferably 4.5-5.5 mg/ml, more preferably 5 mg/ml.

The present invention further provides a formulation comprising the liposomal composition of irinotecan hydrochloride or the liposomal composition of irinotecan hydrochloride prepared by the above preparation method, and a pharmaceutically acceptable carrier and excipient, preferably the formulation is an intravenous injection formulation.

The liposomal composition of irinotecan hydrochloride or the formulation provided by the present invention can be used for treating a tumor or cancer.

The liposomal composition of irinotecan hydrochloride or the formulation provided by the present invention exhibits significantly reduced hematological toxicity (e.g., a significant reduction in the incidence of neutropenia or leukopenia, *etc.*, particularly a significant reduction in the incidence of life-threatening neutropenia) in the subject being treated.

The liposomal composition of irinotecan hydrochloride or the formulation provided by the present invention is capable of reducing the distribution and accumulation of SN-38 in the bone marrow.

The present invention provides a method for treating a tumor or cancer, comprising administering to a subject in need thereof the liposomal composition of irinotecan hydrochloride or the formulation of the present invention.

The present invention provides use of the liposomal composition of irinotecan hydrochloride or the formulation in the manufacture of a medicament for treating a tumor or cancer.

The present invention provides use of the liposomal composition of irinotecan hydrochloride or the formulation in the manufacture of a medicament for significantly reducing the hematological toxicity of irinotecan in the subject being treated.

The present invention provides use of the liposomal composition of irinotecan hydrochloride or the formulation in the manufacture of a medicament for significantly reducing the distribution and accumulation of SN-38 in the bone marrow.

In some embodiments of the present invention, the tumor or cancer preferably includes gastric cancer, pancreatic cancer, lung cancer (e.g., non-small cell lung cancer), colorectal cancer, esophageal cancer, liver cancer, breast cancer, colon cancer, ovarian cancer, and cervical cancer, *etc*. More preferably, the tumor or cancer is selected from advanced solid tumors, including gastric cancer, pancreatic cancer, small cell lung cancer, colorectal cancer, esophageal cancer, liver cancer, and breast cancer, *etc*.

In some embodiments of the present invention, when the composition or formulation is used for treating a tumor or cancer, it exhibits significantly reduced hematological toxicity (e.g., a significant reduction in the incidence of neutropenia or leukopenia, *etc.*, particularly a significant reduction in the incidence of life-threatening neutropenia).

In some embodiments of the present invention, the incidence of ≥ grade 3 (e.g., grade 3/4) neutropenia in the subject being treated is ≤50%, preferably ≤40%, preferably ≤30%, more preferably ≤20%, more preferably ≤10%, more preferably ≤5%, and most preferably 0%.

In some embodiments of the present invention, the incidence of ≥ grade 3 (e.g., grade 3/4) leukopenia in the subject being treated is ≤30%, preferably ≤20%, more preferably ≤10%, more preferably ≤5%, and most preferably 0%.

In some embodiments of the present invention, the subject being treated is a patient with locally advanced or metastatic solid tumors who has failed standard therapy, is intolerant to it, or has no standard therapy available.

In some embodiments of the present invention, the liposomal composition of irinotecan hydrochloride or the formulation is administered in combination with other drugs, said other drugs may be, for example, 5-fluorouracil (5-FU), leucovorin (LV), *etc*.

In some embodiments of the present invention, the administration route of the liposomal composition of irinotecan hydrochloride or the formulation is injection, such as intravenous infusion.

In some embodiments of the present invention, the administration dose of the liposomal composition of irinotecan hydrochloride or the formulation is 40-100 mg/m², preferably 60-90 mg/m², preferably 70 or 80 mg/m².

In some embodiments of the present invention, the liposomal composition of irinotecan hydrochloride or the formulation is administered once every two weeks.

The present invention provides a method for reducing the hematological toxicity of a drug, comprising administering to a subject in need thereof the liposomal composition of irinotecan hydrochloride as described in any one of the above items, the liposomal composition of irinotecan hydrochloride prepared by the preparation method as described in any one of the above items, or the formulation as described in any one of the above items.

The present invention provides a method for reducing the incidence of neutropenia and/or leukopenia, comprising administering to a subject in need thereof the liposomal composition of irinotecan hydrochloride as described in any one of the above items, the liposomal composition of irinotecan hydrochloride prepared by the preparation method as described in any one of the above items, or the formulation as described in any one of the above items.

The present invention provides a method for reducing the incidence of neutropenia and/or leukopenia to ≤50%, comprising administering to a subject in need thereof the liposomal composition of irinotecan hydrochloride as described in any one of the above items, the liposomal composition of irinotecan hydrochloride prepared by the preparation method as described in any one of the above items, or the formulation as described in any one of the above items.

In some embodiments of the present invention, the neutropenia and/or leukopenia is ≥ grade 3 neutropenia and/or leukopenia.

The present invention provides a method for reducing the distribution and accumulation of SN-38 in the bone marrow, comprising administering to a subject in need thereof the liposomal composition of irinotecan hydrochloride as described in any one of the above items, the liposomal composition of irinotecan hydrochloride prepared by the preparation method as described in any one of the above items, or the formulation as described in any one of the above items.

The present invention provides use of a pharmaceutical composition of irinotecan for reducing hematological toxicity, the use comprising administering to a subject in need thereof the liposomal composition of irinotecan hydrochloride as described in any one of the above items, the liposomal composition of irinotecan hydrochloride prepared by the preparation method as described in any one of the above items, or the formulation as described in any one of the above items.

The present invention provides use of a pharmaceutical composition of irinotecan for reducing the incidence of neutropenia and/or leukopenia, the use comprising administering to a subject in need thereof the liposomal composition of irinotecan hydrochloride as described in any one of the above items, the liposomal composition of irinotecan hydrochloride prepared by the preparation method as described in any one of the above items, or the formulation as described in any one of the above items.

The present invention provides use of a pharmaceutical composition of irinotecan for reducing the incidence of neutropenia and/or leukopenia to ≤50%, the use comprising administering to a subject in need thereof the liposomal composition of irinotecan hydrochloride as described in any one of the above items, the liposomal composition of irinotecan hydrochloride prepared by the preparation method as described in any one of the above items, or the formulation as described in any one of the above items.

In some embodiments of the present invention, the neutropenia and/or leukopenia is ≥ grade 3 neutropenia and/or leukopenia.

The present invention provides use of a pharmaceutical composition of irinotecan for reducing the distribution and accumulation of SN-38 in the bone marrow, the use comprising administering to a subject in need thereof the liposomal composition of irinotecan hydrochloride as described in any one of the above items, the liposomal composition of irinotecan hydrochloride prepared by the preparation method as described in any one of the above items, or the formulation as described in any one of the above items.

Compared with the prior art, the present invention has the following beneficial effects:
1. The resulting liposomal formulation of irinotecan hydrochloride has a simple prescription and process, high encapsulation efficiency, good stability, and uniform particle size distribution, facilitating control over its *in vivo* distribution process.
2. Through systematic control of the composition and ratios of the liposomes, the ratio of drug to phospholipid, the composition and concentration of the internal aqueous phase, the mass ratio of phospholipid to ethanol, and the aqueous and organic phases in the liposome preparation method, the resulting liposomal formulation exhibits significantly reduced toxicity, particularly significantly reduced hematological toxicity, and especially a significant reduction in the incidence of neutropenia and leukopenia (particularly neutropenia).
3. The liposome of irinotecan hydrochloride of the present invention, when administered to patients with advanced solid tumors, can significantly reduce the severe hematological toxicity of irinotecan hydrochloride (especially the incidence of neutropenia) and markedly improve patient survival status.

### DETAILED DESCRIPTION OF EMBODIMENTS

The following examples and experimental examples are provided to further illustrate the present invention, but the invention is not limited thereto.

Where specific conditions are not indicated in the examples and experimental examples, they are carried out under conventional conditions or conditions recommended by the manufacturer. Reagents or instruments as employed for which the manufacturer is not specified are conventional products available commercially.

Irinotecan hydrochloride was produced by Fermion. Hydrogenated soybean phospholipids and MPEG2000-DSPE were produced by Lipoid GmbH. Cholesterol was produced by Nippon Fine Chemical Co., Ltd. Ammonium sulfate, disodium hydrogen phosphate, and sodium dihydrogen phosphate were produced by Hunan Jiudian Co., Ltd. Sucrose was produced by Merck KGaA.

### Example 1 Preparation of Irinotecan Hydrochloride Liposomes

**Table 1**

| **Formulation Component** | **Formulation Amount (g)** |
|---|---|
| Irinotecan hydrochloride | 0.5 |
| Hydrogenated soybean phospholipids | 2.295 |
| Cholesterol | 0.773 |
| mPEG2000-DSPE | 0.042 |
| Ethanol | 16 |
| Ammonium sulfate | 2.91 |
| Ethanol phase : aqueous phase (V/V) | 1:5 |

### Preparation Process

1.1 Preparation of an ethanol phase: The prescribed amounts of hydrogenated soybean phospholipids and cholesterol from Table 1 were weighed and dissolved in the absolute ethanol from Table 1 at 60°C. The solution was maintained at the temperature for later use.

1.2 Preparation of an aqueous phase: The prescribed amount of ammonium sulfate from Table 1 was weighed and dissolved in purified water at 60°C to obtain an ammonium sulfate solution with a concentration of 0.22 M. The solution was maintained at the temperature for later use.

1.3 The ethanol phase was injected into the aqueous phase. The liposomes were processed to 95 nm using an extrusion device equipped with two 0.1 µm filters.

1.4 Removal of ammonium sulfate from the external aqueous phase: A 9% sucrose (w/v)-0.1 M phosphate buffer solution (pH=6.5) was prepared as the displacement medium. The external aqueous phase of the liposomes was displaced using a tangential flow ultrafiltration device. Ultrafiltration was stopped when the ammonium ion concentration at the permeation end was detected to be not greater than 0.11 mM, yielding blank liposomes.

1.5 Drug loading: A mixed solution of the prescribed amount of irinotecan hydrochloride and mPEG2000-DSPE was added to the blank liposomes having the ammonium ion gradient difference. The mixture was incubated at 55-60°C for 10 min, and then quenched in an ice bath to obtain the liposomal solution of irinotecan hydrochloride. The solution was sterilized by filtration through a 0.2 µm filter membrane, filled under a nitrogen atmosphere into vials, and sealed. The liposomes had a particle size of 99 nm, an encapsulation efficiency of 97.1%, and an irinotecan hydrochloride concentration of 5 mg/ml.

### Experimental Example 1: Safety Study of Irinotecan Hydrochloride Liposomes in Patients with Advanced Solid Tumors

Test Drug: Irinotecan hydrochloride liposomes; Specification: 10 ml:50 mg; Route of Administration: intravenous drip; Dose: 80 mg/m²; Study Duration: 2 cycles, each cycle lasting 14 days, administered once every two weeks (q2w), administered via intravenous drip over 1.5 h (±10 min) on Day 1 of each cycle.

Control Drug: Conventional irinotecan hydrochloride injection (Trade name: Campto); Specification: 5 ml:0.1 g; Route of Administration: intravenous drip; Dose: 180 mg/m²; Study Duration: 2 cycles, each cycle lasting 14 days, administered once every two weeks (q2w), administered via intravenous drip over 1.5 h (±10 min) on Day 1 of each cycle.

Rationale for Dose Selection of Control and Test Drugs: Preliminary pharmacokinetic studies in human found that the exposure levels of irinotecan hydrochloride liposome group at 80 mg/m² was similar to that of the conventional irinotecan hydrochloride injection group at 180 mg/m².

Patient Inclusion Criteria: Patients with locally advanced or metastatic solid tumors diagnosed histologically (primarily colorectal cancer, pancreatic cancer, gastric cancer, lung cancer, and breast cancer) who had failed standard therapy, or were intolerant to it, or had no standard therapy available, and had at least one measurable lesion according to solid tumor efficacy evaluation criteria.

Enrolled Patients: 12 cases. Among them, 6 in the irinotecan hydrochloride liposome group; and 6 in the conventional irinotecan injection group.

Results: The efficacy of the irinotecan liposome group at 80 mg/m² was comparable to that of the conventional injection at 180 mg/m², but the duration of response (DOR) for disease remission was significantly prolonged compared to that of the conventional injection. Safety test results are shown in Table 2 below. The incidence of grade 3/4 diarrhea was comparable to that of the conventional injection (16.7% vs. 16.7%), while the incidence of grade 3/4 neutropenia and leukopenia was significantly lower than that of the conventional injection (neutrophil count reduction 0% vs. 50%, leukocyte count reduction 0% vs. 33.3%). This suggests that the irinotecan hydrochloride liposomes of the present invention, when administered to patients with advanced solid tumors, can significantly reduce the hematological toxicity of irinotecan, particularly significantly reducing the incidence of neutropenia and markedly improving the patients' survival status.

**Table 2: Human Safety Results of Irinotecan Hydrochloride Liposome Group**

| **Group** | | **Irinotecan Hydrochloride Liposomes** | **Conventional Irinotecan Hydrochloride Injection** |
|---|---|---|---|
| Administration frequency | | q2w | q2w |
| Dose | | 80 mg/m² | 180 mg/m² |
| Number of patients | | 6 | 6 |
| Main adverse event types (grade 3/4) | Diarrhea | **16.7% (1/6)** | **16.7% (1/6)** |
| | Neutrophil Count Reduction | 0 | **50% (3/6)** |
| | Leukocyte Count Reduction | 0 | **33.3% (2/6)** |
| Severe adverse events (SAE) | | 16.7% (1/6) | 50% (3/6) |
| Adverse Events (AE) Leading to Dose Reduction | | 0 | 16.7% (1/6) |
| Adverse Events (AE) Leading to Dosing Suspension | | 16.7% (1/6) | 16.7% (1/6) |
| Adverse Events (AE) Leading to Discontinuation | | 16.7% (1/6) | 33.3% (2/6) |

## Claims

1. A liposomal composition of irinotecan hydrochloride, **characterized in that** the liposomal composition comprises components in the following mass ratios: 1 part of irinotecan hydrochloride, 3.67-5.62 parts of hydrogenated soybean phospholipids, 1.23-2.25 parts of cholesterol, and 0.055-0.200 part of methoxy-terminated poly(ethylene glycol) 2000 distearoylphosphatidylethanolamine (mPEG2000-DSPE); the weight ratio of the hydrogenated soybean phospholipids to cholesterol is 2-4.5:1; the liposomal composition further comprises ammonium sulfate in the internal aqueous phase, and the concentration of ammonium sulfate in the internal aqueous phase is 0.16-0.28 M.

2. The liposomal composition of irinotecan hydrochloride according to claim 1, wherein the concentration of ammonium sulfate in the internal aqueous phase is 0.20-0.24 M.

3. The liposomal composition of irinotecan hydrochloride according to claim 2, wherein the concentration of ammonium sulfate in the internal aqueous phase is 0.22 M.

4. The liposomal composition of irinotecan hydrochloride according to any one of claims 1-3, wherein the liposomal composition comprises components in the following mass ratios: 1 part of irinotecan hydrochloride, 4.13-5.05 parts of hydrogenated soybean phospholipids, 1.23-1.86 parts of cholesterol, and 0.0801-0.150 part of methoxy-terminated poly(ethylene glycol) 2000 distearoylphosphatidylethanolamine (mPEG2000-DSPE); the weight ratio of the hydrogenated soybean phospholipids to cholesterol is 2-3.5:1.

5. The liposomal composition of irinotecan hydrochloride according to claim 4, wherein the liposomal composition comprises components in the following mass ratios: 1 part of irinotecan hydrochloride, 4.13-5.05 parts of hydrogenated soybean phospholipids, 1.39-1.71 parts of cholesterol, and 0.081-0.110 part of methoxy-terminated poly(ethylene glycol) 2000 distearoylphosphatidylethanolamine (mPEG2000-DSPE); the weight ratio of the hydrogenated soybean phospholipids to cholesterol is 2-3.5:1.

6. The liposomal composition of irinotecan hydrochloride according to claim 5, wherein the liposomal composition comprises components in the following mass ratios: 1 part of irinotecan hydrochloride, 4.59 parts of hydrogenated soybean phospholipids, 1.5468 parts of cholesterol, and 0.0840 part of methoxy-terminated poly(ethylene glycol) 2000 distearoylphosphatidylethanolamine (mPEG2000-DSPE).

7. The liposomal composition of irinotecan hydrochloride according to any one of claims 1-6, wherein the liposomal composition of irinotecan hydrochloride is prepared by an ethanol injection method and an ammonium sulfate gradient method, wherein the mass ratio of hydrogenated soybean phospholipids to ethanol in the ethanol phase is 0.1:1-0.5:1, the concentration of ammonium sulfate in the aqueous phase is 0.16-0.28 M, and the volume ratio of the ethanol phase to the aqueous phase is 1:3-1:6.

8. The liposomal composition of irinotecan hydrochloride according to claim 7, wherein the mass ratio of hydrogenated soybean phospholipids to ethanol in the ethanol phase is 0.1:1-0.35:1, and the concentration of ammonium sulfate in the aqueous phase is 0.20-0.24 M.

9. The liposomal composition of irinotecan hydrochloride according to claim 8, wherein the mass ratio of hydrogenated soybean phospholipids to ethanol in the ethanol phase is 0.1:1-0.2:1, and the concentration of ammonium sulfate in the aqueous phase is 0.22 M.

10. The liposomal composition of irinotecan hydrochloride according to any one of claims 1-9, wherein the liposomal composition has a particle size of 90-110 nm, and the particle size D90 corresponding to the cumulative particle size distribution reaching 90% is < 300 nm.

11. The liposomal composition of irinotecan hydrochloride according to claim 10, wherein the particle size D90 corresponding to the cumulative particle size distribution reaching 90% is < 200 nm.

12. The liposomal composition of irinotecan hydrochloride according to claim 11, wherein the particle size corresponding to the cumulative particle size distribution reaching 90% D90 is < 150 nm.

13. The liposomal composition of irinotecan hydrochloride according to any one of claims 1-12, wherein the concentration of irinotecan hydrochloride in the liposomal composition is 4.25-5.75 mg/ml.

14. The liposomal composition of irinotecan hydrochloride according to claim 13, wherein the concentration of irinotecan hydrochloride in the liposomal composition is 4.5-5.5 mg/ml.

15. The liposomal composition of irinotecan hydrochloride according to claim 14, wherein the concentration of irinotecan hydrochloride in the liposomal composition is 5 mg/ml.

16. A method for preparing the liposomal composition of irinotecan hydrochloride according to any one of claims 1-15, **characterized by** comprising the following steps:
a. preparing an ethanol phase: dissolving hydrogenated soybean phospholipids and cholesterol in absolute ethanol at 50-70°C;
b. preparing an aqueous phase: dissolving ammonium sulfate in purified water at 50-70°C to obtain an ammonium sulfate solution with an ammonium sulfate concentration of 0.16-0.28 M;
c. injecting the ethanol phase into the aqueous phase at a volume ratio of 1:3-1:6, and processing the liposomes to 85-150 nm using an extrusion device;
d. removing ammonium sulfate from the external aqueous phase: preparing a phosphate buffer solution at pH 5.0-7.0 as a displacement medium to displace the external aqueous phase of the liposomes, thereby forming an ammonium ion gradient difference of greater than 10³-fold between the internal and external aqueous phases;
e. drug loading: adding a mixed solution of irinotecan hydrochloride and mPEG2000-DSPE to the blank liposomes having the ammonium ion gradient difference, followed by incubation at 55-60°C for 5-30 minutes to obtain the liposomal composition of irinotecan hydrochloride.

17. The method for preparing the liposomal composition of irinotecan according to claim 16, further comprising steps of incubation, quenching, and sterilizing the resulting liposomal solution of irinotecan hydrochloride by filtration through a 0.2 µm filter membrane.

18. The preparation method according to any one of claims 16-17, **characterized in that**:
the dissolution temperature in steps a and b is 60°C; and/or
the volume ratio of the ethanol phase to the aqueous phase in step c is 1:5; and/or
the phosphate buffer solution in step d is a 9% sucrose (w/v)-0.1 mol/L phosphate buffer solution with pH 6.5; and/or
the drug loading time in step e is 10 minutes.

19. A formulation comprising the liposomal composition of irinotecan hydrochloride according to any one of claims 1-15 or the liposomal composition of irinotecan hydrochloride prepared by the preparation method according to any one of claims 16-18, and a pharmaceutically acceptable carrier and/or excipient.

20. The liposomal composition of irinotecan hydrochloride according to any one of claims 1-15, the liposomal composition of irinotecan hydrochloride prepared by the preparation method according to any one of claims 16-18, or the formulation according to claim 19 for use in treating a tumor or cancer, preferably the tumor or cancer comprises gastric cancer, pancreatic cancer, non-small cell lung cancer, small cell lung cancer, colorectal cancer, esophageal cancer, liver cancer, breast cancer, colon cancer, ovarian cancer, and cervical cancer.

21. The liposomal composition of irinotecan hydrochloride according to any one of claims 1-15, the liposomal composition of irinotecan hydrochloride prepared by the preparation method according to any one of claims 16-18, or the formulation according to claim 19 for use in treating a tumor or cancer, wherein the tumor or cancer is selected from advanced or metastatic solid tumors, preferably the advanced or metastatic solid tumors comprise gastric cancer, pancreatic cancer, small cell lung cancer, colorectal cancer, esophageal cancer, liver cancer, and breast cancer.

22. The liposomal composition of irinotecan hydrochloride according to any one of claims 1-15, the liposomal composition of irinotecan hydrochloride prepared by the preparation method according to any one of claims 16-18, or the formulation according to claim 19, which exhibits significantly reduced hematological toxicity during tumor or cancer treatment, for example, significantly reduced hematological toxicity of irinotecan in the subject being treated.

23. The liposomal composition of irinotecan hydrochloride according to any one of claims 1-15, the liposomal composition of irinotecan hydrochloride prepared by the preparation method according to any one of claims 16-18, or the formulation according to claim 19, wherein the incidence of ≥ grade 3 neutropenia in the subject being treated during tumor or cancer treatment is ≤50%.

24. The liposomal composition of irinotecan hydrochloride according to any one of claims 1-15, the liposomal composition of irinotecan hydrochloride prepared by the preparation method according to any one of claims 16-18, or the formulation according to claim 19, which is capable of reducing the distribution and accumulation of SN-38 in the bone marrow.

25. Use of the liposomal composition of irinotecan hydrochloride according to any one of claims 1-15, the liposomal composition of irinotecan hydrochloride prepared by the preparation method according to any one of claims 16-18, or the formulation according to claim 19 in the manufacture of a medicament for treating a tumor or cancer.

26. The use according to claim 25, wherein the tumor or cancer comprises gastric cancer, pancreatic cancer, non-small cell lung cancer, small cell lung cancer, colorectal cancer, esophageal cancer, liver cancer, breast cancer, colon cancer, ovarian cancer, and cervical cancer.

27. The use according to claim 26, wherein the tumor or cancer is selected from advanced or metastatic solid tumors, preferably the advanced or metastatic solid tumors comprise gastric cancer, pancreatic cancer, small cell lung cancer, colorectal cancer, esophageal cancer, liver cancer, and breast cancer.

28. The use according to any one of claims 25-27, wherein the incidence of ≥ grade 3 neutropenia in the subject being treated is ≤50%.

29. A method for treating a tumor or cancer, comprising administering to a subject in need thereof the liposomal composition of irinotecan hydrochloride according to any one of claims 1-15, the liposomal composition of irinotecan hydrochloride prepared by the preparation method according to any one of claims 16-18, or the formulation according to claim 19.

30. The therapeutic method according to claim 29, wherein the tumor or cancer comprises gastric cancer, pancreatic cancer, non-small cell lung cancer, small cell lung cancer, colorectal cancer, esophageal cancer, liver cancer, breast cancer, colon cancer, ovarian cancer, and cervical cancer.

31. The therapeutic method according to claim 30, wherein the tumor or cancer is selected from advanced or metastatic solid tumors, preferably the advanced or metastatic solid tumors comprise gastric cancer, pancreatic cancer, small cell lung cancer, colorectal cancer, esophageal cancer, liver cancer, and breast cancer.

32. The therapeutic method according to any one of claims 29-31, wherein the administration dose of the liposomal composition of irinotecan hydrochloride or the formulation is 40-100 mg/m², preferably 60-90 mg/m², preferably 70 or 80 mg/m².

33. The therapeutic method according to any one of claims 29-32, which has significantly reduced hematological toxicity.

34. The therapeutic method according to any one of claims 29-33, which is capable of significantly reducing the hematological toxicity of irinotecan, including a significant reduction in the incidence of neutropenia or leukopenia, particularly a significant reduction in the incidence of life-threatening neutropenia.

35. The therapeutic method according to any one of claims 29-34, wherein the incidence of ≥ grade 3 (e.g., grade 3/4) neutropenia in the subject being treated is ≤50%.

36. The therapeutic method according to any one of claims 29-35, which is capable of significantly reducing the distribution and accumulation of SN-38 in bone marrow.
